# EUROPEAN PATENT SPECIFICATION

(11) **EP 3 347 476 B1**
(45) Date of publication and mention of the grant of the patent: **31.03.2021**
(21) Application number: 16767164.3
(22) Date of filing: 05.09.2016
(51) Int. Cl.: C12N 15/82

(54) **GENETICALLY ENGINEERED PLANT FIBRES PRESENTING ENHANCED SURFACE PROPERTIES**
GENETISCH VERÄNDERTE PFLANZENFASERN MIT VERBESSERTEN OBERFLÄCHENEIGENSCHAFTEN
FIBRES VÉGÉTALES GÉNÉTIQUEMENT MODIFIÉES PRÉSENTANT DES PROPRIÉTÉS DE SURFACE AMÉLIORÉES

(30) Priority: 11.09.2015 LU 92825
(43) Date of publication of application: 18.07.2018
(73) Proprietor: Luxembourg Institute of Science and Technology (LIST), 4362 Esch/Alzette (LU)
(72) Inventor: GUERRIERO, Gea, 4360 Esch/Alzette (LU)
(74) Representative: Lecomte & Partners
(86) International application number: PCT/EP2016/070797
(87) International publication number: WO 2017/042113

(56) References cited:
- EP-A1- 2 631 296
- WO-A1-2011/089021
- WO-A1-2013/149801
- SZOPA J ET AL: "Chemical composition and molecular structure of fibers from transgenic flax producing polyhydroxybutyrate, and mechanical properties and platelet aggregation of composite materials containing these fibers", COMPOSITES SCIENCE AND TECHNOLOGY, ELSEVIER, UK, vol. 69, no. 14, 1 November 2009 (2009-11-01), pages 2438-2446, XP026665791, ISSN: 0266-3538, DOI: 10.1016/J.COMPSCITECH.2009.06.017 [retrieved on 2009-06-26]
- MICHAEL K. DEYHOLOS ET AL: "Engineering bast fiber feedstocks for use in composite materials", BIOCATALYSIS AND AGRICULTURAL BIOTECHNOLOGY, vol. 3, no. 1, 7 September 2013 (2013-09-07), pages 53-57, XP055250781, ISSN: 1878-8181, DOI: 10.1016/j.bcab.2013.09.001
- Neil Hobson ET AL: "LuFLA1PRO and LuBGAL1PRO promote gene expression in the phloem fibres of flax (Linum usitatissimum)", Plant Cell Reports, 18 January 2013 (2013-01-18), page 517, XP055250791, Berlin DOI: 10.1007/s00299-013-1383-8 Retrieved from the Internet: URL:http://rd.springer.com/article/10.1007 %2Fs00299-013-1383-8#/page-1 [retrieved on 2016-02-17] cited in the application
- GENNADY POGORELKO ET AL: "Post-synthetic modification of plant cell walls by expression of microbial hydrolases in the apoplast", PLANT MOLECULAR BIOLOGY, KLUWER ACADEMIC PUBLISHERS, DORDRECHT, NL, vol. 77, no. 4 - 5, 11 September 2011 (2011-09-11), pages 433-445, XP019965436, ISSN: 1573-5028, DOI: 10.1007/S11103-011-9822-9
- Wróbel-Kwiatkowska, M. et al.: "Engineering of plants for improved fibre qualities" In: Nierstrasz, V.; Cavaco-Paulo, A.: "Advances in Textile Biotechnology", 1 September 2010 (2010-09-01), Woodhead Publishing Ltd, XP009188556, pages 150-170, the whole document, in particular pages 156-160

## Description

### Technical field

The invention is directed to the genetic engineering of plant fibres, notably fibre crops, in order to modify the fibre properties, in particular the surface properties such as hydrophobicity.

### Background art

The use of plant fibres, in particular fibre crops, for the reinforcement of biocomposite is important. An advantage for using plant fibres is that they are renewable, cheap and they raise no health-related issues. However, plant fibres are hygroscopic. Indeed, it is known that when the biocomposites absorb moisture, the plant fibres swell and their subsequent shrinking triggers the formation of cracks. The plant fibres do not bind well with the different resins that are used in this field because of their hydrophilic nature.

An advantage of the fibre crops is that they produce bast fibres. The bast fibres are defined as extraxylary sclerenchymatous elements obtained from the stem cortex of various plants and can be used as reinforcements for polymeric materials. Those fibres are composed primarily of cellulose which potentially has a Young's modulus of about 140 GPa (a value comparable with manmade aramid (Kevlar/Twaron) fibres (Summerscales J. et al., Composites: Part A, 2010, 41, 1329-1335). The plants which are currently attracting most interest are flax, hemp, jute (*Corchorus*), kenaf, sisal, ramie, cotton and nettle. In the automotive industry, it is particularly known that plastics made of hemp fibres are used in the manufacture of different components of the car because those fibres are extremely light and extremely resistant to high pressures. Furthermore, those fibre crops, differently from glass fibres, do not present health-related issue.

In order to make the plant fibres hydrophobic, and subsequently to address the above-mentioned drawback of swelling and shrinking depending on the moisture and the poor binding with the resins, different physico-chemical treatments can be performed. But, unfortunately, those can lead to the alteration of the fibre properties and/or of the fibre morphology.

Amphipathic proteins are known to self-assemble into rodlets to coat the aerial hyphae of some filamentous bacteria and fungi with a hydrophobic sheath. Chaplins, rodlins, streptofactins and hydrophobins are examples of those particular proteins. Those types of proteins, secreted by the filamentous bacteria and fungi, lower the water surface tension to allow aerial growth and cover aerial structures, rendering them hydrophobic.

In the paper entitled "LuFLA1_{PRO} and LuBGAL1_{PRO} promote gene expression in the phloem fibres of flax (*Linum usitatissimum*)" (Hobson H., et al., Plant Cell Rep., 2013, 32, 517-528), the authors described the identification and the isolation of flax gene promoters to direct transgene expression in developing and maturing phloem fibres of flax.

European patent application published EP 2 631 296 A1 relates to a method to produce hydrophobins in plants and microbes. The plants are used as "green" factories for the production of those industrially-relevant proteins. Transgenic plants, transgenic seeds and expression cassette are indeed produced by the described method. The proteins are isolated from the leaves or from the seeds of the plant.

International patent application published WO 2011/089021 A1 describes a method for producing hydrophobic bast fibres according to the preamble of claim 1 and a fibrous plant according to the preamble of claim 10.

### Summary of invention

### Technical Problem

The invention has for technical problem to provide plant fibres which present hydrophobicity, improving the biocomposite properties and without altering the intrinsic properties and morphology of the plant fibres.

### Technical solution

The first object of the invention is directed to a method of producing hydrophobic bast fibres, characterized in that said method is a genetic engineering process of a fibrous plant comprising said bast fibres, comprising the following steps:
a) identification of the bast fibre promoter;
b) amplification of the bast fibre promoter; and
c) preparing a cassette by fusing the bast fibre promoter and at least one gene coding for a surface-active protein, by incorporating SEQ ID NO:1, a β-expansin signal peptide, at the 5' locastion of said at least one gene.

In one embodiment said process further comprises the step (d) of cloning said cassette in a first vector, preferentially the pENTR™/D-TOPO® vector.

In one embodiment, said process further comprises the step (e) of recombining said first vector, preferentially the pENTR™/D-TOPO® vector, into a second vector, preferentially the pEarleyGate 302 vector.

In one embodiment, said step (a) is performed by formation of complementary deoxyribonucleic acid libraries (cDNA libraries) and subsequent high-throughput sequencing (RNA-Seq), said formation and sequencing being preferentially performed by using Illumina® Sequencing Technology.

In one embodiment, said step (a) is performed in the above snap-point, (ASP) part and/or in the below snap-point (BSP) part of the stem of said fibrous plant.

In one embodiment, said step (b) is performed by means of polymerase chain reaction (PCR).

In one embodiment, said surface-active protein is selected from the group of hydrophobins, chaplins, rodlins, and streptofactins, preferentially hydrophobins.

In one embodiment, said fibrous plant is selected from the group of flax, hemp, jute, kenaf, ramie, and nettle, preferentially hemp.

In one embodiment, said process comprises a plant transformation step using *Agrobacterium tumefaciens* GV3101.

The second object of the invention is directed to a bast fibre-producing fibrous plant comprising surface-active proteins, characterized in that said fibrous plant comprises a bast fibre promoter fused with a gene coding a surface-active protein where SEQ ID NO:1, a β-expansin signal peptide, is incorporated at the 5' location of said at least one gene, wherein said plant is obtainable by the genetic engineering process in accordance with the first object of the invention. Said fibrous plant is remarkable in that said fibrous plant comprises surface-active proteins.

In one embodiment, said surface-active proteins are selected from the group of hydrophobins, chaplins, rodlins and streptofactins, preferentially hydrophobins.

In one embodiment, said fibrous plant is selected from the group of flax, hemp, jute, kenaf, ramie, sisal, cotton and nettle, preferentially hemp.

In one embodiment, the surface of said fibrous plant is hydrophobic.

### Advantages of the invention

The invention is particularly interesting in that the genetically modified plants will produce those surface-active proteins, therefore modifying their intrinsic properties, in particular hydrophobicity. As no chemical treatments are involved, no alteration in the fibre morphology will be noticed. Those plants, more specifically fibre crops, even more specifically hemp, might be used in different implementations, such as the manufacture of waterproof paper, self-cleaning (e.g. via lotus-effect), and/or waterproof textile. Other implementations in the biocomposite industry and/or in the green biotechnology will probably follow on from those genetically engineered plants. The automotive industry will also use those fibres with enhanced surface properties.

### Brief description of the drawings

Figure 1 indicates the PCR process between the bast-fibre promoter and the gene for surface-active protein.
Figure 2 indicates the PCR process between the bast-fibre promoter, the gene for surface-active protein and the gene for the β-expansin signal peptide.
Figure 3 depicts the pEarleyGate 302 plasmid (from http://www.snapgene.com/resources/plasmid_files/plant_vectors/pEarleyG ate_302/).

### Description of an embodiment

In order to provide plant fibres which present hydrophobic surface properties without altering the intrinsic mechanical properties and morphology of the plant fibres, the technique of genetic engineering will be employed. The goal is to create transgenic fibre crops. Said transgenic fibre crops are thus capable to secrete those surface-active proteins, in particular hydrophobins but also chaplins, rodlins and streptofactins.

The fibre crops are selected from the group consisting of flax, hemp, jute, kenaf, sisal, ramie cotton and nettle. Hemp can be used as plant of choice, in the light of its wide industrial applications.

To perform the genetic engineering of the fibre crops and, more specifically to achieve the expression of the surface-active proteins in bast fibres, the promoter of the gene expressed in the bast fibres of the fibre crops must be identified. It will be thus necessary to identify marker genes for bast fibre thickening, in order to use their promoters to drive expression of the transgene. It is desirable to express the foreign genes during fibre thickening, to avoid possible interference during the elongation phase of the fibre cells. The preferential expression of the genes during this stage will guarantee that the fibres can carry out water/solute exchanges necessary for turgor pressure maintenance during active elongation.

RNA-Sequencing (RNA-Seq), also called whole transcriptome shotgun sequencing (WTSS), will then be carried out on fibres separated from top and bottom internodes (plants aged 1 month) on the fibre crops, in particular hemp.

A stem of fibre crops can indeed be divided into two zones separated by the snap-point. A first zone is the ASP part, namely the Above Snap-Point part. A second zone is the BSP part, namely the Below Snap-Point.

The separation between top and bottom will enable the identification of genes enriched in two different stages of fibre formation, *i.e.* elongation and thickening, respectively. RNA will be separated from the collected bast fibres (three biological replicates, each consisting of a pool of 8-10 plants showing homogeneous height, stem thickness and number of internodes).

The promoter of the gene of the bast fibres is best marked in the BSP part, because the bottom of the hemp undergoes girth increase (*i.e.* secondary growth), while the top of the hemp elongates rapidly.

In fact, it is preferable to choose genes expressed in the bast fibres coming from the BSP part of the hemp because one does not want to interfere with the elongation of the fibres.

cDNA libraries are thus prepared using the Illumina sequencing technology.

After quality control and normalization, the pooled libraries will be processed using an Illumina sequencing platform (MiSeq, LIST or HiSeq, Genecore platform/EMBL) to achieve 20-30 million 75 base pair paired-end reads.

After quality filtering (>Q30), reads will be processed using the commercially available software CLC Genomics Workbench and mapped using the *C. sativa* cv. Santhica *de novo* transcriptome recently generated by the group at LIST-ERIN.

Then, DNA primers are designed to amplify the promoter of the identified genes, using as template hemp genomic DNA. DNA primers will be used to create the cassette to express, *i.e.* the bast-fibre promoter and the gene for surface-active protein, either hydrophobin or chaplin or rodlin or streptofactin.

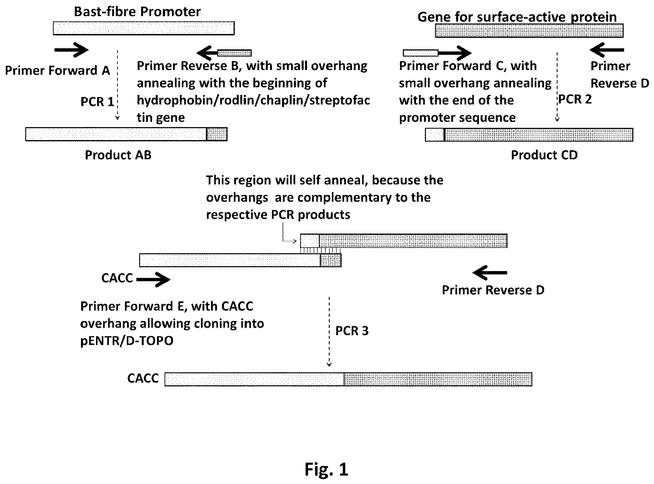

Figure 1 indicates the PCR process between the bast-fibre promoter and the gene for surface-active protein.
- As the bast-fibre promoter is a double-stranded DNA fragment, a forward primer A and a reverse primer B are needed for achieving the first PCR step. The reverse primer B will contain a small overhang adapted for annealing with the beginning of the gene for the surface-active protein. This first PCR step will yield the product AB.
- Similarly, as the gene for the surface-active protein is a double-stranded DNA fragment, a forward primer C and a reverse primer D are needed for achieving the second PCR step. The forward primer C will contain a small overhang adapted for annealing with the end of the promoter sequence. This second PCR step will yield the product CD.
- Then, in the course of the denaturation/annealing step inherent to a third PCR step, the product AB and the product CD will couple together. Those products have been indeed designed to self-anneal in the course of the PCR.
- Then, the third PCR step on the double-stranded DNA fragment obtained by the coupling of AB and CD will be performed. A forward primer E, containing the small overhang with the sequence CACC adapted for cloning the cassette into the pENTR™/D-TOPO® vector will be used. A reverse primer D will be used for the complementary sequence.
- This third PCR step will subsequently yield a cassette which is composed of the bast-fibre promoter fused to the gene coding for the surface-active protein.

This cassette will then be cloned in the pENTR™/D-TOPO® vector and recombined into the pEarleyGate 302 vector.

The pEarleyGate 302 plasmid is a binary vector that will replicate in both *Escherichia coli* and *Agrobacterium tumefaciens* and has left border (LB) and right border (RB) sequences for *Agrobacterium-mediated* T-DNA transfer.

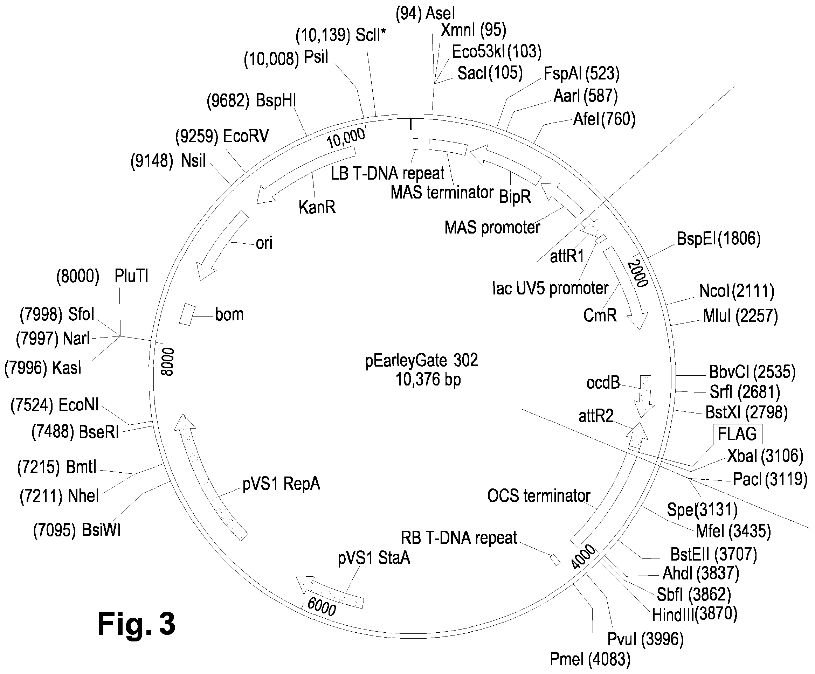

Figure 3 depicts the pEarleyGate 302 plasmid. This vector is known in the literature (Earley K.W., et al., The Plant J., 2006, 45, 616-629).

The region comprised between *att*R1 and *att*R2 will be recombined with the pENTR/D-TOPO vector containing the bast-fibre promoter fused to the gene for the surface-active protein.

Hemp transformation will be performed via *Agrobacterium tumefaciens* GV3101 in accordance with the general knowledge (see notably US patent application published US 2012/0311744 A1). Both hypocotyl explants and calli will be tested for transformation (MacKinnon L., et al., Annual Report of the Scottish Crop Research Institute 2000/2001, eds. W. H. Macfarlane Smith and T. D. Heilbronn (SCRI, Invergowrie, Dundee), 2001, 84-86; Feeney M., et al., In Vitro Cell. Dev. Biol-Plant, 2003, 39, 578-585; Slusarkiewicz-Jarzina A., et al., Acta Biol. Cracov. Ser. Bot., 47, 2005, 145-151; Wang R., et al., Pak. J. Bot., 2009, 41, 603-608; Lata H., et al., In vitro Cell Dev. Biol. Plant, 2009, 45, 12-19; Lata H., et al., Planta Med., 2010, 76, 1629-1633).

In the fungi and in the bacteria, the process of secretion of the surface-active protein is natural. However, in plants, such as fibre crops, this process is not natural. Therefore, to increase the chance of expressing those surface-active proteins into the fibre crops, notably the hemp, the genes are further fused at the 5' with a hemp β-expansin signal peptide **(SEQ ID NO:1).** This gene was previously identified thanks to the *de novo* assembly.

β-expansin is indeed a protein secreted by the plant cells which unlocks the network of wall polysaccharides, thus permitting turgor-driven cell enlargement (Cosgrove D. J., Nature, 2000, 407, 321-326).

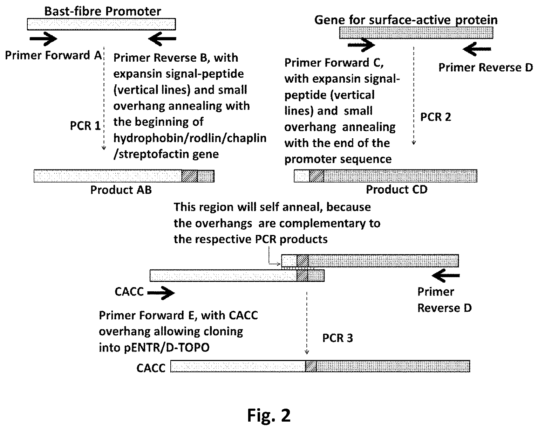

Figure 2 indicates the PCR process between the bast-fibre promoter, the gene for surface-active protein and the gene for the β-expansin signal peptide.
- As the bast-fibre promoter is a double-stranded DNA fragment, a forward primer A and a reverse primer B are needed for achieving the first PCR step. The reverse primer B will contain a small overhang adapted for annealing with the beginning of the gene for the surface-active protein. The reverse primer B will further contain the β-expansin signal peptide sequence.
This first PCR step will yield the product AB.
- Similarly, as the gene for the surface-active protein is a double-stranded DNA fragment, a forward primer C and a reverse primer D are needed for achieving the second PCR step. The forward primer C will contain a small overhang adapted for annealing with the end of the promoter sequence. The forward primer C will further contain the β-expansin signal peptide sequence.
This second PCR step will yield the product CD.
- Then, in the course of the denaturation/annealing step inherent to a third PCR step, the product AB and the product CD will couple together. Those products have been indeed designed to self-anneal in the course of the PCR.
- Then, a third PCR step on the double-stranded DNA fragment obtained by the coupling of AB and CD will be performed. A forward primer E, containing the small overhang with the sequence CACC adapted for cloning the cassette into the pENTR™/D-TOPO® vector will be used. A reverse primer D will be used for the complementary sequence.
- This third PCR step will subsequently yield a cassette which is composed of the bast-fibre promoter fused to the gene coding for the surface-active protein. This cassette will further contain the β-expansin signal peptide sequence.

This cassette will then be cloned in the pENTR™/D-TOPO® vector and recombined into the pEarleyGate 302 vector, depicted in Figure 3.

The same process as it has been designed concerning the cassette devoid of the β-expansin signal peptide sequence will be undertaken in order to generate the genetically engineered plants.
## Claims

1. Method of producing hydrophobic bast fibres, **characterized in that** said method is a genetic engineering process of a fibrous plant comprising said bast fibres, comprising the following steps:
a) identification of the bast fibre promoter;
b) amplification of the bast fibre promoter; and
c) preparing a cassette by fusing the bast fibre promoter and at least one gene coding for a surface-active protein, by incorporating SEQ ID NO:1, a β-expansin signal peptide, at the 5' location of said at least one gene.

2. Method according to claim 1, **characterized in that** said process further comprises the following step:
d) cloning said cassette in a first vector, preferentially the pENTR™/D-TOPO® vector.

3. Method according to one of claims 1-2, **characterized in that** said process further comprises the following step
e) recombining said first vector, preferentially the pENTR™/D-TOPO® vector, into a second vector, preferentially the pEarleyGate 302 vector.

4. Method according to any one of claims 1-3, **characterized in that** said step (a) is performed by formation of complementary deoxyribonucleic acid libraries, and subsequent high-throughput sequencing, like RNA-Seq, said formation and sequencing being preferentially performed by using Illumina® Sequencing Technology.

5. Method according to any one of claims 1-4, **characterized in that** said step (a) is performed in the above snap-point part and/or in the below snap-point part of the stem of said fibrous plant.

6. Method according to any one of claims 1-5, **characterized in that** said step (b) is performed by means of polymerase chain reaction.

7. Method according to any one of claims 1-6, **characterized in that** said surface-active protein is selected from the group of hydrophobins, chaplins, rodlins, and streptofactins, preferentially hydrophobins.

8. Method according to any one of claims 1-7, **characterized in that** said fibrous plant is selected from the group of flax, hemp, jute, kenaf, ramie and nettle, preferentially hemp.

9. Method according to any one of claims 1-8, **characterized in that** said process comprises a plant transformation step using *Agrobacterium tumefaciens* GV3101.

10. Bast fibre-producing fibrous plant comprising surface-active proteins, **characterized in that** said fibrous plant comprises a bast fibre promoter fused with a gene coding a surface-active protein where SEQ ID NO:1, a β-expansin signal peptide, is incorporated at the 5' location of said at least one gene.

11. Fibrous plant according to claim 10, **characterized in that** said surface-active proteins are selected from the group of hydrophobins, chaplins, rodlins and streptofactins, preferentially hydrophobins.

12. Fibrous plant according to any one of claims 10 or 11, **characterized in that** said fibrous plant is selected from the group of flax, hemp, jute, kenaf, ramie, and nettle, preferentially hemp.

13. Fibrous plant according to any one of claims 10-12, **characterized in that** the surface of said fibrous plant is hydrophobic.

## Patentansprüche

1. Verfahren zur Herstellung von hydrophoben Bastfasern, **dadurch gekennzeichnet, dass** es sich bei dem Verfahren um ein gentechnisches Verfahren einer faserhaltigen Pflanze handelt, die die Bastfasern enthält, folgende Schritte umfassend:
a) Identifizierung des Bastfaserpromoters;
b) Amplifikation des Bastfaserpromoters; und
c) Präparation einer Kassette durch Fusionieren des Bastfaserpromoters und mindestens eines Gens, das ein oberflächenaktives Protein codiert, durch Einbau von SEQ ID NO:1, ein β-Expansin-Signalpeptid, an der 5'-Position des mindestens einen Gens.

2. Verfahren nach Anspruch 1, **dadurch gekennzeichnet, dass** das Verfahren außerdem den folgenden Schritt umfasst:
d) Klonierung der Kassette in einen ersten Vektor, vorzugsweise den pENTR™/D-TOPO®-Vektor.

3. Verfahren nach einem der Ansprüche 1 bis 2, **dadurch gekennzeichnet, dass** das Verfahren außerdem den folgenden Schritt umfasst:
e) Rekombination des ersten Vektors, vorzugsweise des pENTR™/D-TOPO® Vektors, in einen zweiten Vektor, vorzugsweise den pEarleyGate 302 Vektor.

4. Verfahren nach einem der Ansprüche 1 bis 3, **dadurch gekennzeichnet, dass** der Schritt (a) durch Bildung von komplementären Desoxyribonukleinsäure-Bibliotheken und anschließende Hochdurchsatz-Sequenzierung, wie RNA-Seq, durchgeführt wird, wobei die Bildung und Sequenzierung vorzugsweise unter Verwendung der Illumina®-Sequenzierungstechnologie durchgeführt wird.

5. Verfahren nach einem der Ansprüche 1 bis 4, **dadurch gekennzeichnet, dass** der Schritt (a) in dem oberhalb des Rasterpunktes liegenden Teil und/oder in dem unterhalb des Rasterpunktes liegenden Teil des Stängels der faserhaltigen Pflanze durchgeführt wird.

6. Verfahren nach einem der Ansprüche 1 bis 5, **dadurch gekennzeichnet, dass** der Schritt (b) mittels Polymerase-Kettenreaktion durchgeführt wird.

7. Verfahren nach einem der Ansprüche 1 bis 6, **dadurch gekennzeichnet, dass** das oberflächenaktive Protein aus der Gruppe der Hydrophobine, Chapline, Rodline und Streptofactine, vorzugsweise Hydrophobine, ausgewählt ist.

8. Verfahren nach einem der Ansprüche 1 bis 7, **dadurch gekennzeichnet, dass** die faserhaltige Pflanze aus der Gruppe von Flachs, Hanf, Jute, Kenaf, Ramie und Nessel, vorzugsweise Hanf, ausgewählt ist.

9. Verfahren nach einem der Ansprüche 1 bis 8, **dadurch gekennzeichnet, dass** das Verfahren einen Schritt der Pflanzentransformation unter Verwendung von *Agrobacterium tumefaciens* GV3101 umfasst.

10. Bastfasern produzierende faserhaltige Pflanze, die oberflächenaktive Proteine umfasst, **dadurch gekennzeichnet, dass** die faserhaltige Pflanze einen Bastfaserpromoter umfasst, der mit einem Gen fusioniert ist, das ein oberflächenaktives Protein codiert, wobei SEQ ID NO:1, ein β-Expansin-Signalpeptid, an der 5'-Position des mindestens einen Gens eingebaut ist.

11. Faserhaltige Pflanze nach Anspruch 10, **dadurch gekennzeichnet, dass** die oberflächenaktiven Proteine aus der Gruppe der Hydrophobine, Chapline, Rodline und Streptofactine, vorzugsweise Hydrophobine ausgewählt sind.

12. Faserhaltige Pflanze nach einem der Ansprüche 10 oder 11, **dadurch gekennzeichnet, dass** die faserhaltige Pflanze aus der Gruppe Flachs, Hanf, Jute, Kenaf, Ramie und Nessel, vorzugsweise Hanf, ausgewählt ist.

13. Faserhaltige Pflanze nach einem der Ansprüche 10 bis 12, **dadurch gekennzeichnet, dass** die Oberfläche der faserhaltigen Pflanze hydrophob ist.

## Revendications

1. Procédé de production de fibres libériennes hydrophobes, **caractérisé en ce que** ledit procédé est un procédé de génie génétique d'une plante fibreuse comprenant des fibres libériennes, comprenant les étapes suivantes de :
a) identification du promoteur de la fibre libérienne ;
b) l'amplification du promoteur de la fibre libérienne ; et
c) préparation d'une cassette par fusion du promoteur de la fibre libérienne avec au moins un gène codant pour une protéine tensioactive, par une incorporation de la SEQ ID NO:1, un peptide signal β-expansine, à la position 5' dudit au moins un gène.

2. Procédé selon la revendication 1, **caractérisé en ce que** ledit procédé comprend en outre l'étape suivante de :
d) clonage de ladite cassette dans un premier vecteur, de préférence le vecteur pENTR™/D-TOPO®.

3. Procédé selon l'une des revendications 1-2, **caractérisé en ce que** ledit procédé comprend en outre l'étape suivante de
e) recombinaison dudit premier vecteur, de préférence le vecteur pENTR™/D-TOPO®, en un second vecteur, de préférence le vecteur pEarleyGate 302.

4. Procédé selon l'une quelconque des revendications 1 à 3, **caractérisé en ce que** ladite étape a) est réalisée par la formation de banques d'acides désoxyribonucléiques complémentaires, de séquençage à haut débit subséquent, comme l'ARN-Seq, ladite formation et ledit séquençage étant réalisés de préférence en utilisant la technologie Illumina®Sequencing.

5. Procédé selon l'une quelconque des revendications 1 à 4, **caractérisé en ce que** ladite étape a) est réalisée dans la partie au-dessus du nœud et/ou dans la partie au-dessous du nœud de la tige de ladite plante fibreuse.

6. Procédé selon l'une quelconque des revendications 1 à 5, **caractérisé en ce que** ladite étape b) est réalisée au moyen d'une réaction en chaîne par polymérase.

7. Procédé selon l'une quelconque des revendications 1 à 6, **caractérisé en ce que** ladite protéine tensioactive est choisie dans le groupe des hydrophobines, chaplins, rodlins et streptofactines, de préférence des hydrophobines.

8. Procédé selon l'une quelconque des revendications 1 à 7, **caractérisé en ce que** ladite plante fibreuse est choisie dans le groupe constitué par le lin, le chanvre, le jute, le kénaf, la ramie et l'ortie, de préférence le chanvre.

9. Procédé selon l'une quelconque des revendications 1 à 8, **caractérisé en ce que** ledit procédé est réalisé par l'intermédiaire de *Agrobacterium tumefaciens* GV3101.

10. Plante fibreuse produisant des fibres libériennes comprenant des protéines tensioactives, **caractérisée en ce que** ladite plante fibreuse comprend un promoteur de fibre libérienne fusionné avec au moins un gène codant pour une protéine tensioactive, dans laquelle SEQ ID NO:1, un peptide signal β-expansine, est incorporée à la position 5' dudit au moins un gène.

11. Plante fibreuse selon la revendication 10, **caractérisée en ce que** lesdites protéines tensioactives sont choisies dans le groupe des hydrophobines, chaplins, rodlins et streptofactines, de préférence des hydrophobines.

12. Plante fibreuse selon la revendication 10 ou 11, **caractérisée** en ce ladite plante fibreuse est choisie dans le groupe constitué par le lin, le chanvre, le jute, le kénaf, la ramie, le sisal et l'ortie, de préférence le chanvre.

13. Plante fibreuse selon l'une quelconque des revendications 10 à 12, **caractérisée en ce que** la surface de ladite plante fibreuse est hydrophobe.


<110> LIST
   Guerriero Gea
<120> Genetically engineered plants presenting enhanced surface properties
<130> sequence listing of PT04134 WO
<160> 1
<210> 1
   <211> 26
   <212> PRT
   <213> Cannabis Sativa L.
<223> Hemp beta-expansin signal peptide
<400> 1

Met Gln Leu Val Arg Ser Val Cys Phe Gly Ala Leu Thr Leu Leu
1               5                   10                  15
Leu Val Phe Lys Tyr Ser Leu Leu Val Asp Ala
16              20                  25